Europäisches Patentamt

European Patent Office

Office européen des brevets

Publication number: **0 026 546**
**B1**

# EUROPEAN PATENT SPECIFICATION

Date of publication of patent specification: **04.09.85**

Application number: **80200910.0**

Date of filing: **26.09.80**

Int. Cl.⁴: **C 07 C 41/03,** C 07 C 43/13, C 07 C 43/23, C 08 G 65/28

Process for reaction of epoxides with organic compounds having an active hydrogen.

Priority: **27.09.79 US 79538**
**27.09.79 US 79496**

Date of publication of application:
**08.04.81 Bulletin 81/14**

Publication of the grant of the patent:
**04.09.85 Bulletin 85/36**

Designated Contracting States:
**BE DE FR GB IT NL SE**

References cited:
**EP-A-0 006 105**
**EP-A-0 018 463**
**EP-A-0 020 867**
**EP-A-0 026 544**
**DE-A-2 628 778**
**US-A-3 240 819**
**US-A-3 972 948**

**CHEMICAL ABSTRACTS, vol. 74, no. 24, June 14, 1971, page 9, abstract 126177j, Columbus, Ohio, USA, K.S. KAZANSKII et al. "Polymerization of ethylene oxide in the presence of alkaline earth alcoholates"**

Proprietor: **UNION CARBIDE CORPORATION**
**Old Ridgebury Road**
**Danbury Connecticut 06817 (US)**

Inventor: **McCain, James Herndon**
**1987 Parkwood Road**
**Charleston West Virginia 25314 (US)**
Inventor: **Foster, Donald Joseph**
**603 39th Street, S.E.**
**Charleston West Virginia 25304 (US)**

Representative: **Urbanus, Henricus Maria, Ir. et al**
**c/o Vereenigde Octrooibureaux Nieuwe Parklaan 107**
**NL-2587 BP 's-Gravenhage (NL)**

Courier Press, Leamington Spa, England.

# 0 026 546

## Description

### Background of the invention

This invention relates to the preparation of reaction products of epoxides with organic compounds having an active hydrogen and, more particularly, to a process for preparing condensation reaction products of an epoxide and organic compound having an active hydrogen with a restricted molecular weight distribution and reduced by-products.

A variety of products such as surfactants, functional fluids, glycol ethers, polyols, and the like are commercially prepared by the condensation reaction of epoxides with organic compounds having an active hydrogen, generally in the presence of an alkaline or acidic catalyst. The types of products prepared and properties thereof depend on the active hydrogen compound, the epoxide, and the number of moles of epoxides employed as well as the catalyst, a mixture of condensation products species being obtained containing different molecular proportions of epoxide. Thus, the reaction products generally obtained have a wide range of molecular weights and of molecular distribution of the epoxide units.

It is generally desirable to restrict the molecular distribution of the mixture to adjacent analogues of the desired product, insofar as possible, but this is quite difficult to control. Acidic catalysts tend to give a narrower molecular distribution than alkaline catalysts, but also contribute to the formation of undesired by-products. Thus, alkaline catalysts are generally used as the more efficient type of catalyst but the molecular distribution in the resulting products are more diffuse.

Heretofore, several methods have been suggested for providing reaction products of an active hydrogen compound and epoxides having a narrower range of molecular weights and molecular distribution of the epoxide units, or which reduce or eliminate the production of undesirable poly(alkylene glycol) and cyclic and straight chain ether by-products. For example, in U.S. Patent 4,112,231 to Weibull et al it is disclosed that the use of certain neutral inorganic fluoborate and perchlorate salts will catalyze the reaction of epoxides with active hydrogen compounds to give products having a narrower molecular distribution and a larger proportion of desired species, in U.S. Patent No. 3,682,849 to Smith et al improved ethoxylated derivatives of $C_{11}$—$C_{18}$ alcohols are prepared by removing unreacted alcohol and lower ethoxylates from the conventionally produced ethoxylate mixture using vapor phase separation techniques; in U.S. Patent 2,870,220 to Carter, a two-stage process is disclosed for preparing monoalkyl ethers of ethylene glycol and polyethylene glycols of more restricted molecular weight range wherein an alkanol and ethylene oxide is reacted in the presence of an acidic catalyst during the first stage and then in the second stage, after removal of acid catalyst and unreacted alkanol, reacting the mixture with ethylene oxide in the presence of an alkali metal alcoholate of the initial alkanol, and in U.K. Patent 1,501,327 to Laemmle et al is disclosed a method of preparing mono- and poly-glycol ethers substantially free of undesired alkylene glycol by-products which involves heating a reaction mixture containing an alkylene oxide and an alcohol in the presence of a catalyst containing alkali or alkaline earth cations wherein some or all of the catalyst is an anhydrous high boiling liquid residue prepared by concentrating the liquid residue left from the same or similar etherification process after removal of the glycol ether product from the reaction mixture. To the best of our knowledge, however, none of the processes or special catalysts disclosed in the art are completely satisfactory in that they require multi-stage procedures or special acid-resistant equipment, give undesirable by products or simply do not provide sufficient control over the molecular weight distribution. It would be highly desirable, therefore, to develop a process wherein the reaction of an epoxide with an organic compound having an active hydrogen could be more readily carried out to prepare products that have a narrow molecular weight distribution of analogue species and contain only small amounts, at most, of undesirable poly(alkylene glycol) and ether by-products.

### Summary of the invention

A process using catalysts, which have been obtained from barium oxide in the manner disclosed above, has been proposed in the copending application 80200908.4 (EP—A—0 026 544) of even date and for that reason it is disclaimed here.

It has been discovered that soluble basic salts of the alkaline earth metals hereinabove described not only catalyze the reaction but also favor a narrower molecular distribution, i.e. a more limited range of molecular species and a larger proportion of the desired species in the reaction product. Moreover, the process of the invention can be carried out in a single stage without the need for special acid-resistant equipment and the products produced thereby have been found, in general, to contain only small amounts of undesired poly(alkylene glycol) and ether by-products.

### Description of the invention

According to the invention reaction products are prepared of an organic compound having an active hydrogen with an epoxide, which reaction is carried out in the presence of compounds of calcium, strontium and/or barium as a catalyst, and the invention is characterized in that products having a narrow molecular weight distribution are prepared by reacting an organic compound selected from primary or secondary saturated alkanols having 1—30 carbon atoms, saturated cycloaliphatic monohydric alcohols having 3—8 carbon atoms, phenyl-substituted monohydric alkanols, polyols having 2—30 carbon atoms, phenols, mono- and dicarboxylic acids and compounds, which contain both amino and hydroxyl groups

2

with an epoxide in the presence of at least a catalytic amount of a basic salt of calcium, strontium and/or barium which is soluble in the reactants and the reaction products, or of a compound of calcium, strontium and/or barium which in situ is converted to such a soluble basic salt, with the exception that the catalyst is not obtained by reacting barium oxide with an alkanol having 1—4 carbon atoms, mixing a polyol or a higher aliphatic, cycloaliphatic or phenyl- substituted aliphatic monohydric alcohol having at least 4 carbon atoms, which is different from the $C_{1-4}$-alkanol with the $C_{1-4}$-alkanol-barium reaction product and removing the $C_{1-4}$-alkanol from the reaction product.

A similar process has been proposed in applicants' pending patent application 80200908.4 (EP—A—0026544) of even date. In that application it is proposed to use a catalyst, prepared from barium oxide in the manner mentioned above and for that reason the use of such catalysts is disclaimed here.

In a preferred embodiment of the process of the invention, a reactive hydrogen compound selected from monohydric alcohols having from 8 to 20 carbon atoms, an alkyl substituted phenol, and a difunctional polypropylene oxide polymer having an average molecular weight in the range of 1000 to 5000 is reacted with an alkylene oxide having 2 to 4 carbon atoms in the presence of a catalytic amount of a basic salt of an alkaline earth metal selected from calcium, strontium, barium and mixtures of the same which is soluble in the reactants and the reaction products.

The reaction may be conducted in a conventional manner, that is, the active hydrogen compound and the catalyst are placed in a reactor, epoxide is added at the reaction temperature until the desired number of moles which may be from about 1 to about 60 or more moles of epoxide per mole of active hydrogen compound have been added, and the product is removed from the reactor and neutralized. The reaction may be conducted in the presence of a solvent, but usually a solvent is not employed.

The temperature at which the reaction proceeds is not narrowly critical and generally products can be made at a reasonable rate of reaction and without decomposition of the product at a temperature between about 50°C and 270°C with a temperature between about 100°C and 200°C being generally preferred. While the pressure of the reaction is not narrowly critical, when low-boiling epoxides such as ethylene oxide and propylene oxide are employed, a pressurized reactor is preferably used.

The product made may be neutralized with a phenol or with any acid that will convert the catalyst to a neutral salt, as for example, acetic acid, carbon dioxide, sulfuric acid and phosphoric acid.

Organic compounds having an active hydrogen atom to which the present invention is applicable may be monohydric alcohols, phenols, polyols, mono- and dicarboxylic acids, and amines.

The monohydric alcohols can be saturated primary and secondary aliphatic alkanols which are straight or branched chain and have from one to thirty carbon atoms and preferably from 8 to 20 carbon atoms. Exemplary of such primary straight chain monohydric alkanols are methanol, ethanol, butanol, pentanol, hexanol, heptanol, octanol, nonanol, decanol, undecanol, dodecanol, tridecanol, tetradecanol, pentadecanal, hexadecanol, octadecanol, heptadecanol, nonadecanol and eicosanol; and of such branched chain or secondary alkanols are isopropyl alcohol, 2-ethylhexanol, sec-butanol, iso-butanol, 2-pentanol, 3-pentanol, iso-octanol, sec-octanol, isodecanol, 2-methyl-1-nonanol, 2-methyl-1-undecanol, 2-methyl-1-dodecanol, 2-methyl-1-tetradecanol, 4-tetradecanol and 6-heptadecanol. Mixtures of such alkanols including commercially available alkanols which normally comprise mixtures of alkanols are also suitable. Particularly suitable are linear and branched primary alkanols and alkanol mixtures such as are produced by the "Oxo" reaction of normal $C_3$—$C_{20}$ olefins.

The process of the invention is also applicable to saturated cycloaliphatic monohydric alcohols having 3—8 C-atoms, including for example, cyclohexanol, cyclopentanol, cycloheptanol, cyclopropanol and cyclooctanol, as well as phenyl-substituted monohydric alcohols such as benzyl alcohol, phenylethyl alcohol, and phenylpropyl alcohol.

Applicable phenols include, for example, phenol, alkylphenols having 12 to 22 carbon atoms, such as p-methylphenol, p-ethylphenol, p-butylphenol, p-heptyphenol, p-nonylphenol, dinonylphenol, and p-decylphenol. The aromatic radicals may contain other conventional substituents such as halogen atoms.

The polyols to which the present invention is applicable can have from two to thirty carbon atoms and may have from two to six hydroxyl groups including, for example, glycerine, glycols such as ethylene glycol, propylene glycol, butylene glycol, pentylene glycol, hexylene glycol, heptylene glycol, neopentylene glycol, decylene glycol, diethylene glycol, triethylene glycol, propylene glycol, dipropylene glycol, tripropylene glycol, pentaerythritol, galactitol, sorbitol, mannitol, erythritol, trimethylolethene and trimethylolpropane.

Also suitable are difunctional propylene oxide polymers having a molecular weight of 1000 to 5000, and preferably 1700 to 4100. The propylene oxide polymers having a molecular weight of 1000 to 5000 contains from 17 to 86 oxypropylene units in the molecule. These compounds are well known being generally obtained by polymerization of propylene oxide or by the addition of propylene oxide to lower molecular compounds with 2 to 6 carbon atoms containing at least 2 reactive hydrogen atoms.

The carboxylic acids that may be used include, for example, acetic acid, propionic acid, butyric acid, pentanoic acid, hexanoic acid, heptanoic acid, octanoic acid, nonanoic acid, decanoic acid and undecanoic acid, terephthalic acid, and fatty acids such as stearic acid, oleic acid, and tall oil acids and unsaturated acids such as acrylic, methacrylic and crotonic acids.

The amines to which the present invention is applicable are organic compounds which contain both amino and hydroxyl groups including, for example, monoethanolamine, N-methylmonoethanolamine, N-ethylmonoethanolamine, N-(n-propyl) monoethanolamine, N-(isopropyl) monoethanolamine, N-(n-butyl) monoethanolamine, diethanolamine, N,N-dimethylethanolamine, N,N-diethylethanolamine, N,N-di(n-propyl) ethanolamine, N,N-di(isopropyl) ethanolamine, N,N-di(n-butyl) ethanolamine and triethanol amine. Also useful can be propanolamines such as monopropanolamine, dipropanolamine, and tripanolamine, as well as the amino-phenols such as p-aminophenol, m-aminophenol, and o-aminophenol.

The epoxides which are applicable in accordance with the invention can be any epoxide having from two to thirty carbon atoms, and preferably alkylene oxides, having from 2 to 4 carbon atoms. Exemplary epoxides include alkylene oxides such as ethylene oxide; 1,2-propylene oxide; 1,2- and 2,3-butylene oxide; 1,2-pentylene oxide; 1,2-hexylene oxide; 1,2-octylene oxide; and 1,2-decylene oxide; and mixtures of the same; epoxidized fatty alcohols derived from fatty oils such as epoxidized soybean fatty alcohols and epoxidized linseed oil fatty alcohols; cycloalkylene epoxides including, for example, cyclohexene oxide, cyclopentene oxide, cycloheptene oxide; aromatic epoxides such as styrene oxide and 2-methylstyrene oxide; and hydroxy- and halogen-substituted epoxides such as glycidol, epichlorohydrin and epibromhydrin.

The number of moles of alkylene oxides employed according to the present invention may vary widely depending on the reactive hydrogen compound to be adducted and the particular application for which the product is to be employed. In general, in the preparation of surface active agents, 2 to 60 and even more moles of alkylene oxide per mole of reactive hydrogen compound may be used. Insofar as propylene oxide and/or butylene oxide are used in combination with ethylene oxide, the molar ratio to ethylene oxide to propylene—or butylene oxide may be from 1:0.03 to 1:1.

In accordance with the process of the invention, the reaction of an epoxide with an active hydrogen containing compound is catalyzed by the presence of a basic salt of alkaline earth metal selected from calcium, strontium, barium and mixtures of the same which are soluble in the reactants and the reaction products produced thereby. These basic alkaline earth metal salts may be calcium, strontium, and barium alkoxides, amides phenoxides and the mutual reaction products of an alkaline earth metal hexammoniate, an olefin oxide and an organic nitride such as disclosed in U.S. Patent 2,969,402 to Hill et al. Preferably, the metal alkoxides and phenoxides have alcohol and phenol moieties which are the same or similar to the reactive hydrogen compound reactant of the oxyalkylation reaction. Also suitable are calcium, strontium, and barium compounds, such as the salts thereto which are convertible to soluble basic salts, in situ, during the alkoxylation reaction. Exemplary of such salts include calcium chloride, calcium sulfate, calcium cyanide and barium bromide

Basic salts of alkaline earth metals that are suitable for use in accordance with the invention and their method of preparation are known. However, a preferred soluble basic salt of alkaline earth metal for use as a catalyst in the invention, particularly in the reaction of an epoxide and various monohydric alcohol and polyol reactive hydrogen compounds is disclosed in copending European patent application 80200911.8 (EP—A—0026547). Said copending application relates to calcium, strontium and barium alkoxides, in which the alcohol moiety is the same or similar to the alcohol or polyol reactive hydrogen compound reactant component. Such alkoxides have been found to be a particularly efficacious catalyst in various embodiments of the present invention.

Such metal alkoxides are, in general, prepared by a two step process. In the first step of the process, for example, calcium, strontium and barium, or their hydrides or acetylides may be reacted with a lower alkanol having 1 to 7 carbon atoms. The concentration of metal in the lower alcohol may vary from 0.01 to 20 percent. In the second step, the lower alkanol metal alkoxide reaction product is reacted with a polyol or a higher aliphatic, cycloaliphatic or phenyl-substituted aliphatic monohydric alcohol having at least 4 and preferably from 8 to 30 carbon atoms, which is the same or similar to the reactive hydrogen compound to be reacted with the epoxide, thereby preparing a metal alkoxide of the reactive hydrogen compound which is a soluble basic salt having catalytic activity. The lower alkanol introduced with the lower metal alkoxide is removed from the final metal alkoxide reaction product by any separation means that retains the catalytic activity of the alkaline earth metal alkoxide, with distillation being generally preferred.

Alternatively, the alkaline earth metal alkoxide of a lower alkanol prepared in the first step may be added to a reaction mixture comprising a reactive hydrogen compound and epoxide wherein the reactive hydrogen compound is a polyol or higher monohydric alcohol having at least 4 and preferably from 8 to 30 carbon atoms and the soluble basic salt of the alkaline earth metal formed in situ has the catalytic activity desired.

The amount of catalyst used in accordance with the invention is not narrowly critical and a catalytic effect has been noted with only a small amount thereof being present. In general, the catalyst concentration can vary from 0.001 percent to 10 percent by weight of calcium, strontium, and barium based on the weight of active hydrogen compound. Concentrations of alkaline earth metal within the range from 0.05 percent to 5.0 percent by weight of active hydrogen compound are usually preferred. The reaction rate, however, is dependent on both temperature and catalyst concentration and to achieve a given rate, more catalyst is required at a low temperature than at a high temperature.

The invention will become more clear when considered together with the following examples which are set forth as being merely illustrative of the invention and which are not intended, in any manner, to be limitative thereof. Unless otherwise indicated, all parts and percentages are by weight. Pressures in bar are gauge values.

4

# 0 026 546

Example 1

A mixture of 25 grams (0.19 moles) of calcium ethoxide (prepared by the reaction of calcium metal and ethanol) and 1000 grams (4.7 moles) of a mixture of $C_{12}$ to $C_{15}$ primary alkanols (60% branched, 40% normal isomers) available under the tradename LIAL-125 from Liquichemica Italia was heated at 90°C under high vacuum in a stirred flask distilling off the ethanol and then transferred to a steel, 9.5 liter autoclave equipped with a stirrer, an automatic temperature controller, and an automatic feed controller. The autoclave was heated to 110°C, pressurized to 1.4 bar with nitrogen and then to 4.1 bar with ethylene oxide. By automatic control, 1685 grams (38.3 moles) of ethylene oxide was fed to the autoclave at 110°C over a period of 2 1/2 hours. After cookout to 2.3 bar, the product (2717 grams) was cooled, drained from the reactor, and neutralized to a pH of 7 with acetic acid in a stirred flask.

In a second run, 8.2 grams (0.15 moles) of potassium hydroxide in 1000 grams of the mixture of $C_{12}$ to $C_{15}$ primary alkanols hereinabove described was charged into the 9.5 liter autoclave and heated to 110°C under 4.1 bar pressure as in run 1. 1685 grams (38.3 moles of ethylene oxide was then fed to the autoclave and reacted with the alcohol at 110°C over a period of 0.65 hours. After cookout, the product (2707 grams) was cooled, drained from the reactor and neutralized to a pH of 7 with acetic acid.

Gel permeation chromatography, a standard technique for determining the molecular weight distribution of polymers and surfactants was used to evaluate the reaction products. A comparison of the widths of gel permeation peaks at their half-heights, all run at constant conditions, is a measure of the relative broadness of the molecular weight distribution of the polymers or surfactants. When the performance of the instrument is calibrated with standards of known molecular weights, it is possible to define the molecular weight range represented by the peak width at half height.

Gel permeation chromatography results on the products of run #1 and #2 hereof are reported in Table I, below.

TABLE I

| Catalyst | Gel permeation chromatography results | |
|---|---|---|
| | Peak width at one-half height (cc) | Molecular weight range at one-half height |
| Basic Calcium Alkoxide (Run #1) | 2.9 | 360—820 |
| Potassium Hydroxide (Run #2) | 4.4 | 320—1000 |

The catalytic effect using the basic calcium alkoxide and potassium hydroxide is apparent from the above results. Although the catalytic activity of the basic calcium alcoxide is less than that of potassium hydroxide, the more favorable molecular distribution of the reaction products obtained with this catalyst as compared to potassium hydroxide is apparent.

Pour point (ASTM D-9766) and cloud point (ASTM D-2024, 1% solution) values were also determined for the products prepared in runs #1 and #2. The product of run #1 using the basic calcium alkoxide catalyst had a pour point of 17°C and a cloud point of 60°C, both of which values are more favourable than the pour point of 23°C and cloud point of 55°C determined for the products produced in run #2.

Example 2

A mixture of a modified calcium amide containing 5.75 grams of calcium (0.14 moles) prepared according to the method disclosed in U.S. Patent 2,969,402 to Hill et al. and 1000 grams (4.7 moles) of the mixture of $C_{12}$ to $C_{15}$ primary alkanols of Example 1 was charged to the autoclave of Example 1 and 1687 grams (38.3 moles) of ethylene oxide was reacted with it using the procedure of Example 1 at 110°C and 4.1 bar over a period of 1.8 hours. After cookout, the product (2673 grams) was cooled, drained from the reactor, and neutralized to a pH of 7 with acetic acid. The calcium containing basic catalyst was soluble in the reaction mixture drained from the reactor but precipitated therefrom when neutralized with acetic acid. Gel permeation chromatography results on the product formed are reported in Table 2, below.

Using the procedure and autoclave hereinabove described, a mixture of 6.21 grams (0.16 moles) of sodium hydroxide in 1000 grams (4.7 moles) of the mixture of $C_{12}$ to $C_{15}$ primary alkanols of Example 1 was reacted with 1685 grams (38.3 moles) of ethylene oxide at 110°C and 4.1 bar over a period of 0.75 hours. After cookout, the product (2721 grams) was cooled and neutralized with acetic acid. The sodium hydroxide catalyst was soluble in the reaction mixture and did not precipitate when neutralized with acetic acid. Gel permeation chromatography results on the product formed are reported in Table 2, below.

5

# 0 026 546

## TABLE 2
### Gel permeation chromatography results

| Catalyst | Peak width at one-half height (cc) | Molecular weight range at one-half height |
|---|---|---|
| Modified Basic Calcium Amid | 2.9 | 360—840 |
| Sodium Hydroxide | 4.2 | 340—1050 |

The catalytic effect using the modified basic calcium amide and sodium hydroxide materials is apparent, though the catalytic activity of the basic calcium salt is somewhat less than that of sodium hydroxide. However, the more favorable molecular distribution of the reaction products obtained with the basic calcium salt material as compared to sodium hydroxide is apparent from the above results.

Example 3

Run #1: A mixture of calcium ethoxide (0.072 moles) in ethanol (140 cc) prepared by the reaction of calcium metal and ethanol was added to 500 grams (2.7 moles) of 1-dodecanol and heated in a stirred flask at 110°C under high vacuum to remove the ethanol. The resulting mixture was charged to the autoclave of Example 1 where 770 grams (17.5 moles) of ethylene oxide were added, and, using the procedure of Example 1, reacted at 140°C and 4.1 bar. After completion of the reaction, a portion of the product (507 grams) was removed from the autoclave and neutralized with acetic acid. The calcium containing basic catalyst was soluble in the reaction mixture removed from the autoclave until neutralized with acetic acid. Gel permeation chromatography results for the reaction product are reported in Table III, below.

To the remaining portion of the reaction product in the autoclave was added 767 grams (13.7 moles) of propylene oxide at 140°C and 4.1 bar. A portion of the resulting product (525 grams) was removed from the autoclave and neutralized. The molecular weight of the product was determined to be 832.

Run #2: Using the autoclave and procedure hereinabove described, a mixture of potassium hydroxide (0.072 moles) and 500 grams (2.7 moles) of 1-dodecanol was stripped in a stirred flask at 110°C and high vacuum and then charged to the autoclave. Ethylene oxide (757 grams—17.2 moles) was reacted with the charge in the autoclave at 140°C and 4.1 bar. After completion of the reaction, a portion of the product (559 grams) was removed from the autoclave and neutralized with acetic acid. The gel permeation chromatography results are reported in Table III, below.

To the remaining portion of the reaction mixture in the autoclave were added 701 grams (12.5 moles) of propylene oxide at 140°C and 4.1 bar. After completion of the reaction, a portion of the product (555 grams) was removed from the autoclave and neutralized. The molecular weight of the product was determined to be 926.

## TABLE III
### Gel permeation chromatography results

| Catalyst | Peak width at one-half height (cc) | Molecular weight range one half height |
|---|---|---|
| Run #1 Calcium Salt | 2.9 | 290—640 |
| Run #2 Potassium Hydroxide | 4.5 | 270—930 |

The catalytic effect using the basic calcium salt and potassium hydroxide in the reaction with both ethylene oxide and propylene oxide is apparent. While the catalytic activity of the basic calcium salt was found to be lower than that of potassium hydroxide, the narrower molecular weight distribution of the reaction products as compared to potassium hydroxide was very favorable.

Example 4

The autoclave and procedure of Example 1 were employed in this Example during the following reactions.

Run #1: Calcium ethoxide (0.5 moles) in 767 grams of ethanol was added to 853 grams (11.5 moles) of butanol and the ethanol was removed by distillation yielding a butanol solution containing 0.84 moles of calcium per 1000 grams of solution.

A quantity of butanol (795 grams—10.7 moles) containing calcium prepared above and charged to the autoclave. Propylene oxide (3710 ml.—66 moles) was added to the autoclave at 115°C under 4.1 bar at a rate that maintained a constant pressure in the autoclave. After completion of the reaction, the product (3063 grams) was neutralized with phosphoric acid. The molecular weight and molecular weight range results are reported in Table IV, below.

Run #2: Using the procedure of Run #1 hereof, a 50/50 weight percent mixture of ethylene oxide and

6

propylene oxide was reacted with butanol. The molecular weight and molecular weight range results are reported in Table IV.

Run #3: Using the procedure of Run #1 hereof, a 75/25 weight percent mixture of ethylene oxide and propylene oxide was reacted with butanol and the results are reported in Table IV.

Runs #3, 4, and 5 were run using the alkylene oxide and butanol reactants of Runs #1, #2 and #3 hereof respectively except that in each of these runs, preformed potassium butylate was employed as the catalyst. Results determined for the products produced in Runs 4, 5, and 6 are reported in Table IV. The catalyst compositions employed in tests runs 1, 2, and 3 were soluble in the reaction mixtures prior to neutralization.

The catalytic effect using the soluble basic calcium alkoxide and potassium butylate materials is apparent. It is also shown that products having a significantly narrower molecular weight distribution are obtained with the basic calcium salt catalyst as compared with the potassium butylate catalyst.

TABLE IV

| Run | Catalyst | Epoxide | Molecular weight | Gel permeation chromatography results | |
| --- | --- | --- | --- | --- | --- |
| | | | | Peak width at one-half height (cc) | Molecular weight range at one-half height |
| 1 | Calcium | propylene oxide | 398 | 2.8 | 300—570 |
| 2 | Calcium | 50/50 ethylene oxide/ propylene oxide | 425 | 4.2 | 290—800 |
| 3 | Calcium | 75/25 ethylene oxide/ propylene oxide | 924 | 3.2 | 650—1400 |
| 4 | Potassium | propylene oxide | 394 | 3.7 | 240—600 |
| 5 | Potassium | 50/50 ethylene oxide/ propylene oxide | 488 | 4.3 | 300—820 |
| 6 | Potassium | 75/25 ethylene oxide/ propylene oxide | 926 | 3.4 | 560—1300 |

Example 5

A 100 cc "rocking" autoclave reactor equipped with automatic temperature control means is used in this Example. In each test run of this Example, 15.0 grams (0.075 moles) of lauric acid containing 0.14 moles of the catalyst noted below per mole of lauric acid and 33.0 grams (0.75 moles) of ethylene oxide are charged into the reactor, the reactor is closed and then heated at 110°C and autogenous pressure for 20 hours. Upon completion of the reaction, unreacted ethylene oxide is stripped from the reaction mixture and collected in a dry ice trap.

The catalyst is prepared by reacting an appropriate amount of calcium, strontium, or barium metal with ethanol at reflux in a flask protected from atmospheric moisture and then mixing 20 grams of the metal ethoxide solution with sufficient lauric acid to form a solution containing 0.14 moles of the appropriate metal per mole of lauric acid; after removal of the ethanol. The ethanol is removed from the lauric acid solution by distillation at 100°C and 267 Pa.

Run #1: In this run, calcium ethoxide is prepared using 0.23 moles of calcium metal per 1000 grams of ethanol and then 32 grams of lauric acid is mixed with 20 grams of the calcium ethoxide to give a solution of 0.14 moles of calcium per mole of lauric acid.

Run #2: 20 grams of strontium ethoxide prepared in the proportion of 0.36 moles per 1000 grams of ethanol is mixed with 50.2 grams of lauric acid to give a solution containing 0.14 moles of strontium per mole of lauric acid.

Run #3: Barium ethoxide is prepared using 0.38 moles of barium metal per 1000 grams of ethanol and then 52.8 grams of lauric acid is mixed with 20 grams of the barium ethoxide to give a solution containing 0.14 moles of barium per mole of lauric acid.

In each of the runs, substantially all of the ethylene oxide is reacted with lauric acid to give a reaction product that is a mixture of poly(oxyethylene) laurate, poly(oxyethylene) dilaurate, and poly(oxyethylene).

Example 6

A mixture of 1.5 liters of calcium ethoxide in ethanol (prepared by reacting 28 grams (0.70 moles) of calcium metal with 1.5 liters of ethanol at reflux) and 600 grams (6.5 moles) of glycerine is heated under vacuum to remove the ethanol.

Five hundred and sixty five grams (6.14 moles of glycerine) of the solution containing glycerine and basic salt of calcium is charged into a 7.5 liter steel autoclave reactor equipped with an automatic temperature controller, an automatic pressure and reactant feed controller, and circulating means for the reactants. The autoclave is heated to 117°C, pressurized to 0.83 bar with nitrogen and then to 48.3 bar with propylene oxide. Over a 2 hour period, 365 grams (6.3 moles) propylene oxide is fed to the autoclave while maintaining the temperature at 117°C. The temperature of the autoclave is then raised to 135°C and an additional 3390 grams (58.4 moles) of propylene oxide is added over a period of 5 hours while maintaining the temperature at 135°C and a constant pressure. After all the propylene oxide is added, the autoclave is held at 135°C for one additional hour to achieve complete reaction after which the reactor is cooled and the reaction mixture is discharged and analyzed.

The reaction product is a clear solution which is determined to have a hydroxyl number of 238 from which a molecular weight of 707 is calculated assuming a functionality of 3.

Example 7

A mixture of 123 grams of calcium ethoxide in ethanol (prepared by reacting calcium metal with ethanol at reflux) and 510 grams (5.5 moles) of phenol are heated to 55°C under 665 Pa to distill off the ethanol. The resulting solution contains 0.14 moles of calcium per 100 grams of phenol (0.6 weight percent calcium).

Five hundred and twenty grams of the phenol solution prepared above (contains 5.5 moles phenol) is charged to the autoclave reactor of Example 1 which is heated to 140°C and pressurized to 4.1 bar using the procedure of Example 1. Over a period of 0.18 hours, 242 grams (5.5 moles) of ethylene oxide is fed to the autoclave. After cookout to a constant pressure (about 1 hour) the reaction mixture is cooled and a sample of 114 grams is drained from the reactor, neutralized with phosphoric acid and analyzed for molecular weight. Before neutralization the reaction product is a clear solution.

The 648 grams of reaction mixture (4.6 moles) remaining in the autoclave are reheated to 140°C and brought to a pressure of 4.1 bar with ethylene oxide. Over a period of 0.3 hours, 208 grams (4.7 moles) of ethylene oxide are fed to the reactor. After cookout to a constant pressure (about 40 minutes) the reaction mixture is cooled and a sample of 119 grams of reaction product is drained from the reactor, neutralized with phosphoric acid and anaylzed for molecular weight. The reaction mixture is a clear solution before neutralization.

The reaction mixture remaining in the autoclave (737 grams—4.2 moles) is reheated to 140°C and brought to a pressure of 4.1 bar with ethylene oxide. Over a period of 0.23 hours, 177 grams (4.0 moles) of ethylene oxide is fed to the autoclave. After cookout to a constant pressure (about 1 hour), the reaction mixture is cooled and drained from the reactor, neutralized with phosphoric acid and analyzed. The reaction product is a clear solution before neutralization.

The reaction product samples taken during the three steps of the process are determined to have the following molecular weights:

9

**0 026 546**

First step: molecular weight of 142
Second Step: molecular weight of 177
Final Step: molecular weight of 219

Example 8

In a series of test runs, 1-dodecanol was reacted with ethylene oxide in the autoclave reactor of Example 1 using the procedure of Example 1 to evaluate the catalysts noted in Table II, below. In each run, 500 grams (2.7 moles) of 1-dodecanol containing 0.10 to 0.12 moles per liter of catalyst was charged to the autoclave reactor and reacted with approximately 6.7 moles of ethylene oxide at 140°C, except as noted, and 4.1 bar. The products were neutralized with phosphoric acid, filter, and analyzed. The analytical results are reported in Table II and Table III, below.

The catalysts used were prepared as follows: For the potassium catalyst, potassium hydroxide was added to 1-dodecanol, and then water was removed at 110°C and 1333. For the calcium, strontium, and barium catalysts, the metal was first reacted under reflux with excess ethanol or methanol to make a 0.5 mole per liter solution. The alkoxide solution in lower alcohol (ethanol or methanol) was added to 1-dodecanol and lower alcohol any water present was removed at 110°C and 1333 Pa.

10

TABLE II

| Catalyst | Concentra-tion weight percent | Reaction rate (a) | Molecular weight | Moles of ethylene oxide | Appearance | Pour point °C (b) | Cloud point °C (c) | Polyethylene Glycols % (d) |
|---|---|---|---|---|---|---|---|---|
| potassium hydroxide | 0.47 | 330 | 480 | 6.7 | slight haze | 15 | 52 | 0.1 |
| potassium hydroxide | 0.40 | 300 | 479 | 6.7 | clear | 15 | 57 | 0.4 |
| calcium ethoxide I | 0.48 | 82 | 481 | 6.7 | clear | 13 | 53 | nil |
| calcium ethoxide II | 0.48 | 85 | 496 | 7.0 | clear | 13 | 58 | nil |
| strontium ethoxide | 0.97 | 130 | 482 | 6.7 | clear | 10,5 | 52 | 0.1 |
| barium ethoxide | 1.5 | 360 | 488 | 6.9 | slight haze | 10,5 | 54 | 0.1 |
| calcium methoxide | 0.48 | no reaction at 140°C | — | — | — | — | — | — |
| calcium methoxide | 0.48 | 68(e) | 472 | 6.5 | haze | | 57 | 0.3 |

(a)moles of ethylene oxide per mole of catalyst per hour
(b)ASTM D-9766
(c)1% solution ASTM D-2024
(d)by thin layer chromatography
(e)at 170°C

0 026 546

TABLE III

Gel permeation chromatography results

| Catalyst | Concentration (weight percent) | Peak width at one-half height (cc) | Molecular weight range at one-half height |
|---|---|---|---|
| Potassium hydroxide | 0.48 | 4.5 | 270—930 |
| Calcium ethoxide I | 0.48 | 2.9 | 290—640 |
| Strontium ethoxide | 0.97 | 3.1 | 290—690 |
| Barium ethoxide | 1.51 | 3.3 | 280—690 |
| Calcium methoxide | 0.48 | 3.3 | 280—690 |

The results show that soluble basic salts of barium, strontium, and calcium catalyze the oxyethylation of the monohydric alcohol to give reaction products having a narrower molecular weight distribution and lower pour points compared to products made with potassium hydroxide.

Example 9

A mixture of 150 grams of barium ethoxide (prepared by the reaction of barium metal and ethanol in the proportions of 0.60 mols Ba/1000 grams of ethanol) and 550 grams of nonylphenol (2.5 moles) is heated at 110°C under high vacuum to distill off the ethanol. The solution thus formed contains 0.16 moles Ba/1000 gms of organic compound (2.2 weight percent of barium).

Using the autoclave reactor of Example 1, 500 grams (2.3 moles of nonylphenol) of the nonylphenol solution prepared above is charged to the reactor which is heated to 140°C under 4.1 bar pressure. Over a period of 0.4 hour, 1062 grams (24 moles) of ethylene oxide is fed to the reactor. After cookout to a constant pressure (about 1 hour), the reaction mixture is cooled, drained from the reactor, neutralized, and analyzed. The catalyst compound is in solution with the reaction mixture until neutralization.

The reaction product which is an ethylene oxide adduct of nonylphenol is determined to have a molecular weight of 682, a pour point of 4.3°C and a cloud point (0.5% aqueous solution) of 65°C.

**Claims**

1. A process for the preparation of reaction products of an organic compound having an active hydrogen with an epoxide, which reaction is carried out in the presence of compounds of calcium, strontium and/or barium as a catalyst, characterized in that products having a narrow molecular weight distribution are prepared by reacting an organic compound selected from primary or secondary saturated alkanols having 1—30 carbon atoms, saturated cycloaliphatic monohydric alcohols having 3—8 carbon atoms, phenyl-substituted monohydric alkanols, polyols having 2—30 carbon atoms, phenols, mono- and dicarboxylic acids and compounds, which contain both amino and hydroxyl groups with an epoxide in the presence of at least a catalytic amount of a basic salt of calcium, strontium and/or barium which is soluble in the reactants and the reaction products, or of a compound of calcium, strontium and/or barium which in situ is converted to such a soluble basic salt, with the exception that the catalyst is not obtained by reacting barium oxide with an alkanol having 1—4 carbon atoms, mixing a polyol or a higher aliphatic, cycloaliphatic or phenyl substituted aliphatic monohydric alcohol having at least 4 carbon atoms, which is different from the $C_{1-4}$-alkanol with the $C_{1-4}$-alkanol-barium reaction product and removing the $C_{1-4}$-alkanol from the reaction product.

2. The process of claim 1, in which the catalyst is a soluble basic alkaline earth metal salt selected from calcium, strontium and barium alkoxides, calcium, strontium and barium amides, calcium, strontium and barium phenoxides, and the mutual reaction product of a calcium, strontium or barium hexammoniate, an olefin oxide and an organic nitride.

3. The process of claim 1, in which the organic compound is selected from monohydric alkanols, phenols, and polyols.

4. The process of claim 1 wherein said reactive hydrogen compound is a member selected from monohydric alcohols having from 8 to 20 carbon atoms, an alkyl substituted phenol, and a difunctional polypropylene oxide polymer having an average molecular weight in the range of 1000 to 5000.

5. The process of claim 4, wherein the organic starting hydrogen compound is a primary alkanol.

**0 026 546**

6. The process of any one of claims 1 to 5 wherein said epoxide is an alkylene oxide having from 2 to 4 carbon atoms or mixtures thereof.

7. The process of any one of claims 1 to 6 in which the catalyst is selected from calcium, strontium and barium alkoxides.

8. The process of any one of claims 1 to 7 in which the catalyst concentration is within the range of 0.001 percent and 10 percent by weight of alkaline earth metal based on the organic starting hydrogen compound.

9. The process of claim 1, in which the organic starting hydrogen compound is a polyol having from two to thirty carbon atoms and from two to six hydroxyl groups.

## Patentansprüche

1. Verfahren zur Herstellung von Reaktionsprodukten einer organischen, aktiven Wasserstoff enthaltenden Verbindung mit einem Epoxid, wobei die Reaktion in Anwesenheit von Verbindungen des Calciums, Strontiums und/oder Bariums als Katalysator durchgeführt wird, dadurch gekennzeichnet, daß Produkte mit einer engen Molekulargewichtsverteilung hertestellt werden, indem man eine organische Verbindung, ausgewählt aus primären oder sekundären gesättigten Alkanolen mit 1 bis 30 Kohlenstoffatomen, gesättigten cycloaliphatischen einwertigen Alkoholen mit 3 bis 8 Kohlenstoffatomen, phenylsubstituierten einwertigen Alkanolen, Polyolen mit 2 bis 30 Kohlenstoffatomen, Phenolen, Mono- und Dicarbonsäuren und Verbindungen, die sowohl Amino- als auch Hydroxylgruppen enthalten, mit einem Epoxid in Anwesenheit mindestens einer katalytischen Menge eines basischen Salzes von Calcium, Strontium und/oder Barium, das in den Reaktionsteilnehmern und den Reaktionsprodukten löslich ist, oder einer Verbindung des Calciums, Strontiums und/oder Bariums, die in situ in ein solches lösliches basischen Salz umgewandelt wird, umsetzt, mit der Ansnahme, daß der Katalysator nicht erhalten wurde, indem man Bariumoxid mit einem Alkanol mit 1 bis 4 Kohlenstoffatomen umsetzt, ein Polyol oder einen höheren aliphatischen, cycloaliphatischen oder phenylsubstituierten aliphatischen einwertigen Alkohol mit mindestens 4 Kohlenstoffatomen, der von dem $C_{1-4}$ Alkanol verschieden ist, mit dem $C_{1-4}$-Alkanol-Bariumreaktionsprodukt mischt und das $C_{1-4}$-Alkanol vom Reaktionsprodukt entfernt.

2. Verfahren nach Anspruch 1, in welchem der Katalysator ein lösliches basisches Erdalkalimetallsalz, ausgewählt aus Calcium-, Strontium- und Bariumalkoxiden, Calcium-, Strontium- und Bariumamiden, Calcium-, Strontium- und Bariumphenoxiden und dem gegenseitigen Reaktionsprodukt aus einem Calcium-, Strontium- oder Bariumhexammoniat, einem Olefinoxid und einem organischen Nitrid, ist.

3. Verfahren nach Anspruch 1, in welchem die organische Verbindung ausgewählt ist aus einwertigen Alkanolen, Phenolen und Polyolen.

4. Verfahren nach Anspruch 1, in welchem die reaktionsfähige Wasserstoffverbindung eine Verbindung, ausgewählt aus einwertigen Alkoholen mit 8 bis 20 Kohlenstoffatomen, einem alkylsubstituierten Phenol und einem difunktionellen Polypropylenoxidpolymerisat mit einem durchschnittlichen Molekulargewicht im Bereich von 1000 bis 5000, ist.

5. Verfahren nach Anspruch 4, welchem die organische wasserstoffhaltige Ausgangsverbindung ein primäres Alkanol ist.

6. Verfahren nach einem der Ansprüche 1 bis 5, in welchem das Epoxid ein Alkylenoxid mit 2 bis 4 Kohlenstoffatomen oder eine Mischung derselben ist.

7. Verfahren nach einem der Ansprüche 1 bis 6, in welchem der Katalysator ausgewählt ist aus Calcium-, Strontium- und Bariumalkoxiden.

8. Verfahren nach einem der Ansprüche 1 bis 7, in welchem die Katalysatorkonzentration im Bereich von 0,001 Gew.-% und 10 Gew.-% Erdalkalimetall, bezogen auf die organische wasserstoffhaltige Ausgangsverbindung, liegt.

9. Verfahren nach Anspruch 1, in welchem die organische wasserstoffhaltige Ausgangsverbindung ein Polyol mit 2 bis 30 Kohlenstoffatomen und 2 bis 6 Hydroxylgruppen ist.

## Revendications

1. Procédé de préparation de produits de réaction d'un composé organique ayant un atome d'hydrogène actif avec un époxyde, réaction qui est conduite en présence de composés de calcium, de strontium et/ou de baryum comme catalyseur, caractérisé en ce que des produits ayant une distribution étroite de poids moléculaire sont préparés par réaction d'un composé organique choisi entre des alcanols saturés primaires ou secondaires ayant 1 à 30 atomes de carbone, des alcools monohydroxyliques cycloaliphatiques saturés ayant 3 à 8 atomes de carbone, des alcanols monohydroxyliques à substituant phényle, des polyols ayant 2 à 30 atomes de carbone, des phénols, des acides monocarboxyliques et dicarboxyliques et des composés qui contiennent à la fois des groupes amino et hydroxyle, avec un époxyde en présence d'au moins une quantité catalytique d'un sel basique de calcium, strontium et/ou baryum qui est soluble dans les corps réactionnels et dans les produits de réaction, ou d'un composé de calcium, strontium et/ou baryum qui est converti in situ en un tel sel basique soluble, sauf que le catalyseur n'est pas obtenu par réaction d'oxyde de baryum avec un alcanol ayant 1 à 4 atomes de carbone, mélange d'un polyol ou d'un alcool monohydroxylique aliphatique supérieur, cycloaliphatique ou aliphatique à

13

# 0 026 546

substituant phényle ayant au moins 4 atomes de carbone, qui est différent de l'alcanol en $C_1$ à $C_4$, avec le produit de réaction entre alcanol en $C_1$ à $C_4$ et baryum et élimination de l'alcanol en $C_1$ à $C_4$ du produit de réaction.

2. Procédé suivant la revendication 1, dans lequel le catalyseur est un sel de métal alcalino-terreux basique soluble choisi entre des alcoolates de calcium, strontium et baryum, des amidures de calcium, strontium et baryum, des phénylates de calcium, strontium et baryum et le produit de réaction mutuelle d'un hexammoniate de calcium, de strontium ou de baryum, d'un oxyde d'oléfine et d'un nitrure organique.

3. Procédé suivant la revendication 1, dans lequel le composé organique est choisi entre des alcanols monohydroxyliques, des phénols et des polyols.

4. Procédé suivant la revendication 1, dans lequel le composé porteur d'hydrogène réactif est choisi dans le groupe comprenant des alcools monohydroxyliques ayant 8 à 20 atomes de carbone, un phénol à substituant alkyle et un polymère difonctionnel qui est un polypropylèneoxyde ayant un poids moléculaire moyen dans l'intervalle de 1000 à 5000.

5. Procédé suivant la revendication 4, dans lequel le composé organique de départ porteur d'hydrogène est un alcanol primaire.

6. Procédé suivant l'une quelconque des revendications 1 à 5, dans lequel ledit époxyde est un oxyde d'alkylène ayant 2 à 4 atomes de carbone ou des mélanges de cet oxyde.

7. Procédé suivant l'une quelconque des revendications 1 à 6, dans lequel le catalyseur est choisi entre des alcoolates de calcium, strontium et baryum.

8. Procédé suivant l'une quelconque des revendications 1 à 7, dans lequel la concentration du catalyseur se situe dans l'intervalle de 0,001 à 10% en poids de métal alcalino-terreux sur la base du composé organique de départ porteur d'hydrogène.

9. Procédé suivant la revendication 1, dans lequel le composé organique de départ porteur d'hydrogène est un polyol ayant deux à trente atomes de carbone et deux à six groupes hydroxyle.